(19) Europäisches Patentamt

European Patent Office

Office européen des brevets

(11) **EP 4 491 119 A1**

(12) **EUROPEAN PATENT APPLICATION**

(43) Date of publication:
**15.01.2025 Bulletin 2025/03**

(21) Application number: **23185262.5**

(22) Date of filing: **13.07.2023**

(51) International Patent Classification (IPC):
*A61B 5/243* (2021.01)    *A61B 5/318* (2021.01)
*A61B 5/349* (2021.01)    *A61B 5/366* (2021.01)
*A61B 5/355* (2021.01)    *A61B 5/00* (2006.01)

(52) Cooperative Patent Classification (CPC):
**A61B 5/243;** A61B 5/7203

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Biomagnetik Park Holding GmbH
21149 Hamburg (DE)**

(72) Inventors:
• **PARK, Jai-Wun
21149 Hamburg (DE)**
• **KIM, Jung-Sun
Seoul 03722 (KR)**
• **WESSEL, Niels
10115 Berlin (DE)**

(74) Representative: **Stüven, Ralf
RGTH Patentanwälte PartGmbB
Kirchenhang 32b
21073 Hamburg (DE)**

(54) **METHOD FOR THE MAGNETOCARDIOGRAPHIC CHARACTERIZATION OF AT LEAST A PORTION OF A HEARTBEAT CYCLE**

(57) The invention relates to a method for the magnetocardiographic characterization of at least a portion of a heartbeat cycle of a heart of a subject. Magnetocardiographic parameter obtained by the method can, for example, be used for the prediction of a risk for sudden cardiac death, SCD, in a subject.

**Fig. 2**

**Description**

The invention relates to a method for the magnetocardiographic characterization of at least a portion of a heartbeat cycle of a heart of a subject, which can, inter alia, be advantageously used in a method for predicting a risk for sudden cardiac death in a subject. The invention further relates to a magnetocardiographic system, and a data carrier.

Magnetography is widely used in the field of medicine as a diagnostic tool for diseases of the brain or the heart. Magnetographic methods for investigating the brain or heart of a subject are known as magnetoencephalography (MEG) and magnetocardiography (MCG), respectively. Magnetographic apparatus measure very weak biomagnetic fields generated by the electric activity of tissue, e.g., heart or brain tissue. Magnetography has various advantages over other diagnostic techniques. The recording is contactless and is therefore not influenced by the conductivity of the body. Most importantly, magnetography does not require any form of radiation or contrast media and, furthermore, is non-invasive.

Magnetocardiography is particularly useful in the examination of a myocardial dysfunction, for example, coronary artery disease (CAD). CAD is an inflammatory process that initially leads to non-obstructive and eventually obstructive atherosclerotic plaques. The process is exacerbated by various genetic and environmental cardiovascular risk factors [2]. A life-threatening circulatory disturbance (ischemia) occurs when these atherosclerotic plaques of the coronary arteries become severely obstructive. CAD may be accompanied by symptoms such as dyspnoea and chest pain. Both obstructive and non-obstructive stenoses can lead to acute circulatory disturbances of the heart. This can lead to acute plaque rupture, thrombosis and myocardial infarction.

CAD and other causes of myocardial ischemia still remain the most common cause of death in industrialized countries. Although chest pain is a common symptom in patients with myocardial ischemia, only about 4% of patients who present to a doctor with chest pain actually have an acute coronary syndrome (ACS). Nearly 850,000 patients underwent diagnostic cardiac catheterization in Germany in 2019, but only about 50% required interventional or cardiac surgery consequences (32nd German Heart Report 2020).

In patients with known CAD or a high pre-test probability of CAD, invasive (coronary) angiography is performed through catheterization. Angiography is recommended for pre-test probability (PTP) between 15% and 50% and allows anatomically accurate morphological visualization of the coronary arteries. The sensitivity is 95-99% which is significantly better than its specificity (64-83%). Some disadvantages are possible contrast media allergy (iodine), contrast media-induced nephropathy/hyperthyroidism, and radiation exposure. Electrocardiographic exercise stress testing followed by potential noninvasive imaging (coronary computed tomography angiography, CCTA) is warranted in patients with clinical symptoms and suspected cardiac cause [4]. Other gold standard methods or techniques that are suggested in the 2019 ESC guidelines CCS such as fraction flow reserve (FFR) or positron emission tomography (PET) are either invasive or have a potential for negative side effects.

Whether a noninvasive diagnostic test like electrocardiography (ECG) is appropriate in case of CAD is determined by the CAD pre-test probability. Statistically, these tests are only meaningful in patients with values between 15% and 85%. Clinical scores (for example SYNTAX Score) including age, sex, cardiovascular risk factors, electrocardiogram (ECG) changes, or elevated heart-specific enzymes (troponin) can be used to determine statistically whether patients have a low, intermediate, or high PTP of CAD [3]. Individually, chest pain, angina-like symptoms, shortness of breath on exertion, and left arm pain are indicative of potential acute myocardial infarction. In patients with suspected CAD further functional or anatomical testing is indicated. Current international guidelines recommend that the choice of test should be based on the individual's PTP of disease. Based on locally available technologies and physician's experience, this may include a coronary CT scan, nuclear imaging tests like PET or single photon emission computed tomography (SPECT), cardiac magnetic resonance imaging (MRI), exercise ECG or stress echocardiography.

Exercise ECG has been a standard clinical method due to its wide availability, low financial expense, and the lack of exposure to radiation. Therefore, it is still the standard examination procedure in community practices and hospitals, and the one of the basic functional tests in suspected CAD [5]. An ECG stress test for CAD is considered positive if there is at least 1 mm of horizontal or downward ST-segment depression. In addition, ST-segment elevation greater than 1 mm is a strong indication of significant ischemia. However, a meta-analysis of 24,074 patients in 147 studies found that the ECG stress test for the detection of CAD had a sensitivity of only 68% and a specificity of 77% [6]. The sensitivity for the detection of stenosing CAD is only 45-50%. Further, there is generally lower diagnostic reliability in women and limited diagnostic significance in patients undergoing digitalis, nitrate, or β-blocker therapy. In addition, in case of an ST-segment depression at rest of 0-1 mm, only with a pre-test probability $\leq$ 30% can a negative finding exclude CAD with sufficient probability (< 15%).

Therefore, the 2019 German national guideline on chronic CAD classifies stress ECG only as a second-line diagnostic method, when imaging modalities are not available locally and the pre-test probability is between 15% and 30%. In contrast, diagnostic imaging such as CT angiography (CTA), stress echocardiography, myocardial perfusion SPECT, or stress MRI, are now recommended by national as well as international guidelines when chronic stable CAD is suspected, mainly because these methods not only have a higher diagnostic sensitivity but are also feasible in patients not being able to undergo an exercise ECG.

Sudden cardiac death (SCD) is defined as an unexpected death or cardiac arrest due to a cardiovascular cause that occurs rapidly mostly due to lethal ventricular arrhythmias in the setting of underlying CAD [8]. Despite major advances in CAD treatment and the use of implantable cardioverter defibrillators (ICDs) for SCD prevention, SCD remains a major public health problem estimated to account for up to 20% of all deaths. In the last decades, CAD mortality declined markedly, but there were large disparities in the magnitude of the decline, resulting in a shift in the distribution toward out-of-hospital and non-CAD deaths [9]. SCD preventive strategies are lacking in those low-risk individuals without established heart disease that comprise the largest proportion of SCDs.

Approximately 80% of individuals who suffer SCD have CAD [10]. Besides CAD as a known risk factor there are other factors used in identifying individuals at risk for SCD, such as age, hypertension, left ventricular hypertrophy, intraventricular conduction block, elevated serum cholesterol, glucose intolerance, decreased vital capacity, smoking, relative weight, and heart rate [11]. Another group of high-risk patients for SCD is those with hereditary ion channel or myocardial defects, such as a long QT syndrome (LQTS) or short QT syndrome (SQTS), hypertrophic cardiomyopathy (HCM), and arrhythmogenic right ventricular dysplasia (ARVD) [12]. All risk groups for SCD most likely share a common feature of impaired intracellular calcium homeostasis [13]. The main causes of death in heart failure patients, declining cardiac pump function and arrhythmias, have both been linked to disrupted $Ca^{2+}$ homeostasis in cardiac muscle cells [14].

It is an object of the present invention to provide an improved, non-invasive method with the aid of which medically relevant parameters can be obtained that can, for example, advantageously be used to improve the prediction of a risk for sudden cardiac death.

In a first aspect the invention provides a method for the magnetocardiographic characterization of at least a portion of a heartbeat cycle of a heart of a subject, the method comprising:

a) acquiring magnetic field data with at least two magnetic field sensors measuring at least one component of a biomagnetic field at at least two different positions above the heart of the subject for a duration of at least a portion of one heartbeat cycle, the portion covering at least the QRS complex and the T wave;

b) selecting a first and a second reference heartbeat cycle time defining the beginning and the end of an evaluation interval, the evaluation interval covering at least the QRS complex and the T wave;

c) calculating, from the magnetic field data acquired during the evaluation interval, a reference signal, the reference signal being the root-mean-square (RMS) value of the magnetic field data of the at least two magnetic field sensors calculated for each sample acquired during the evaluation interval;

d) calculating, from the magnetic field data acquired during the evaluation interval, a monopolarity index, Mi, of the acquired magnetic field data, the monopolarity index being the normalized sum of the magnetic field data of the at least two magnetic field sensors for each sample acquired during the evaluation interval;

e) calculating, from the magnetic field data acquired during the evaluation interval, or from the monopolarity index, Mi, calculated in step d, a monopolarity index signal, MI, of the acquired magnetic field data, the monopolarity index signal resulting from the elementwise multiplication of the reference signal calculated in step c above with the monopolarity index calculated in step d above; and

f) calculating an average value of the MI and RMS values for at least a fraction of the evaluation interval;

wherein steps c to f are computer-implemented.

The method of the invention provides magnetocardiographic parameters that can advantageously be used in the context of heart diseases, e.g., coronary artery disease. Based on the surprising finding that magnetography (MG), in particular magnetocardiography (MCG), can be used for the detection and/or characterization of cellular, i.e., membranous or cytosolic ionic currents in a tissue or organ of a living subject, the method according to the first aspect of the invention is able to provide, from measured magnetic field data, potentially medically relevant parameters for use in a variety of medical methods, for example, in finding a medical diagnosis or in a method for the prediction of a specific risk relating to or resulting from a pathological condition, disorder or dysfunction of the tissue or organ, e.g. heart muscle tissue. The method of the first aspect of the invention has been found to be particularly useful for providing at least one magnetocardiographic parameter that can be used for the prediction of a subject's risk to encounter sudden cardiac death (SCD). In particular, the method of the first aspect of the invention is useful for the prediction of a risk of a subject having or being suspected to have coronary artery disease (CAD), in particular, acute coronary syndrome (ACS).

The terms "cellular ionography" or "magnetoionography" (MIG), if used herein, relate to the detection and characterization of individual cellular ionic currents, i.e., ion currents (fluxes) on a cellular level. This includes membranous cellular ion currents, i.e., ion currents passing the cell membrane, e.g., $Na^+$, $Ca^{2+}$ or $K^+$ ion currents flowing into the cell across the cell membrane via ion channels or out of the cell across the cell membrane via ion channels, or ion currents moving along the cell membrane, and intracellular ion currents not passing the cell membrane, e.g., intracellular $Ca^{2+}$ currents across the sarcoplasmic/endoplasmic reticulum membrane. Without wishing to bound by theory, it is assumed that localized, subsarcolemmal $Ca^{2+}$ release (LCR) via ryanodine receptors (RyRs) during diastolic depolarization reflect rhythmic intracellular $Ca^{2+}$ cycling that does not require the concomitant membrane depolarization (s. [21]). Such "subcellular ionic currents" remain hidden from electrocardiographic measurement methods due to potential shielding by the cell membrane. The term also encompasses, for example, apparent ion currents along the cell membranes as well as intracellular $Ca^{2+}$ transients via RyR2. Unless stated otherwise or clear from the context, the terms "cellular ion flux" or "cellular ion current", as used herein, encompass membranous and intracellular ion fluxes. In particular, the term relates to the detection and characterization of individual ionic currents involved in or occurring during the action potential (AP) at a cell membrane of a living cell.

The terms "radially directed ion flux" or "radial ion flux" relate to ion fluxes (currents) across the cell membrane. The direction of these radial currents (repolarization currents and injury currents) is perpendicular to the cell fiber direction. The term encompasses ion fluxes apparently moving radially in the myocardium, for example, in the direction from subendocardial to subepicardial. In relation to the sensor plane of a magnetographic sensor arrangement, e.g., a magnetocardiographic sensor arrangement, of a plurality of sensors, the term "radial" can also mean in the direction of the anteroposterior axis with the result that solely one magnetic pole is kept by the XY sensor plane.

The terms "tangentially directed ion flux" or "tangential ion flux" relate to ion fluxes (currents) apparently moving along the membranes or along the cell fiber length, for example, from the atrioventricular node (AV node) to the apex of the heart, "apparently" because the propagation of depolarization or repolarization from cell fiber end to cell fiber end via gap junctions appears as a movement of electric charges from cell to cell. In relation to the sensor plane of a magnetographic sensor arrangement, e.g., a magnetocardiographic sensor arrangement, of a plurality of sensors, the term "tangential" can also mean in a direction perpendicular to the anteroposterior axis.

The term "sample" in relation to the acquisition of magnetic field data refers to a single measuring or reading step resulting in a measured value, e.g., the magnetic flux density, at a discrete point in time. A sample thus includes a set of magnetic field data simultaneously acquired by a given number of magnetic field sensors at a given point in time. If, for example, magnetic field data are simultaneously read by 64 of magnetic field sensors over a period of one second at a sampling rate of 500 Hz, 500 samples each covering the magnetic field data of the 64 sensors would result.

The term "magnetic field data" refers to data representing physical properties of a magnetic field. The term encompasses the terms magnetic flux density and magnetic field gradient (the gradient of a magnetic field measured simultaneously at two points in space). Preferably the term relates to the magnetic field gradient.

The term "plurality" as used herein, for example, in relation to a number of magnetic field sensors used to measure magnetic field data, or to a number of different spatial positions (measuring points) above the heart at which magnetic field data are measured, relates to two or more items or elements, e.g., two or more magnetic field sensors or positions. The term "plurality" in relation to magnetic field sensors thus refers to two, three, four, five, six, seven, eight, nine ten or more, e.g., 64, 128 or more magnetic field sensors. In relation to the positions at which magnetic field data are measured, the term relates to two, three, four, five, six, seven, eight, nine ten or more, e.g., 64, 128 or more positions (measuring points).

The term "monopolarity" in relation to a magnetic field generated, for example, by the heart, relates to the degree to which a magnetic field deviates from an essentially dipolar shape, i.e., the degree of a shift from an essentially dipolar shape to a more monopolar shape. In particular, the monopolarity represents a measure for a distribution of positive and negative magnetic field sensor signals deviating from a dipolar pattern. A perfect dipolar shape of a magnetic field would result in an axially symmetric isocontour map plotted from measured data of a plurality of sensors, e.g., axial gradiometers, positioned directly above the magnetic field source and oriented in the z-axis using standard heart coordinates. A shift to a more monopolar shape, i.e., a magnetic field having a higher monopolarity, would result in an axially asymmetric isocontour map, where, for example, the majority of the sensors of a plurality of magnetic field sensors positioned directly above the magnetic field source would measure negative or positive values, and/or where the sum of (absolute) negative values is greater than the sum of the positive values, or vice versa. An example of a magnetic field having an essentially "monopolar" shape would, for example, be a magnetic field where all of a plurality of magnetic field sensors positioned directly above the magnetic field source measure only positive or only negative values. It is to be noted here that the term "essentially monopolar magnetic field" or "monopolar magnetic field" does not denote a magnetic monopole, but a magnetic field where one pole (negative or positive) is distributed over a larger area, e.g., of heart tissue, such that no distinct peak (e.g., minus or plus peak) can be determined. This effect occurs when the direction of the cardiac currents is such that one of the magnetic poles is not visible to the magnetic field sensors. The monopolarity can, for example, be determined by taking magnetic field data measured by magnetic field sensors at a specific point in time or over a specific time interval, and comparing, for example, the number of sensors with negative and positive data and/or comparing the magnitudes of the negative or positive data. An inherent offset of the sensors is, of course, taken into account. A perfectly dipolar magnetic field has no (or zero) monopolarity, whereas a magnetic field for which, for example, a larger number of sensors out of a plurality of sensors measuring the magnetic field measures a positive instead of a negative value, or vice versa, has a specific monopolarity (e.g., greater than zero). In particular, the terms "magnetic field having an essentially monopolar shape" or "essentially monopolar magnetic field" relates to a magnetic field where all of a plurality of magnetic field sensors measure either positive or negative values at a given point in time or over a given time interval. The preferably automatic quantification of the monopolarity of a magnetic field may be implemented in different ways. The terms "monopolarity score" or "monopolarity index" ($M_i$) are used to refer to a quantified value representing the monopolarity.

The term "dipolarity" in relation to a magnetic field generated, for example, by the heart, relates to the degree to which a magnetic field has a dipolar shape, i.e., a shape with two magnetic field extrema. A perfect dipolar shape would result in an axially symmetric isocontour map plotted from measured data of a plurality of sensors positioned directly above the magnetic field source and oriented in, for example, the z-axis using standard heart coordinates. The terms "dipolarity score" or "dipolarity index" are used to refer to a quantified value representing the dipolarity.

The terms "monopolarity index signal", MI, and "dipolarity index signal", DI, are used for a calculated value obtained by weighting the monopolarity or dipolarity index with a reference value, e.g., the root means square of magnetic field data acquired during an evaluation interval.

The term "evaluation interval" as used herein refers to a time interval within the heartbeat cycle, whose magnetic field data is at least partially to be used for an evaluation and/or calculation steps.

The term "first and second reference heartbeat cycle time" refers to two different points in time defining the beginning and the end of an evaluation interval, i.e., a section of a heartbeat cycle, and the magnetic field data acquired during this section.

The term "root mean square" (RMS) in relation to, for example, magnetic field data, refers to the square root of the arithmetic mean of the squares of a set of measured values, e.g., magnetic field data values. In particular, if the term is used in relation to a section of the heartbeat, e.g., the T wave, the term relates to the arithmetic mean of the squares of a set (sample) of magnetic field data acquired simultaneously by a plurality of magnetic field sensors at a point in time.

The term "average value" refers to the arithmetic mean calculated for a set of values.

The terms "heartbeat cycle", "heartbeat", "cardiac cycle" or "cardiocycle" refer to the rhythmic sequence of contraction and relaxation of the heart resulting from the electrophysiological activity of heart muscle cells (cardiomyocytes). One "heartbeat cycle" comprises an atrial systole, a ventricular systole, an atrial diastole, and a ventricular diastole.

The term "sinus rhythm" relates to the rhythmic sequence of contractions of the heart during the cardiac cycle (heartbeat) involving depolarization and repolarization of the atria and ventricles. In the sinus rhythm, depolarization of the cardiac muscle begins at the sinoatrial node (also called sinus node or SA node) situated in the upper part of the wall of the right atrium, is conducted through the atrioventricular node (AV node) between the atria and ventricles to the bundle of His, the bundle branches (Tawara branches) and the Purkinje fibers. Repolarization of the ventricles ends the cycle. Depolarization and repolarization of the atria and ventricles are reflected by three typical waves or wave complexes on a typical electrocardiogram (ECG), P-wave, QRS complex and T-wave. In the ECG, the P-wave is usually considered to represent atrial depolarization, the QRS complex the depolarization of the right and left ventricles, and the T-wave the repolarization of the ventricles.

Terms like "QRS complex", "T wave", etc. relating to sections of the heartbeat cycle are also used in relation to magnetocardiograms. Although originally created for electrocardiography the terms for describing specific sections of an electrocardiogram (e.g., P wave, "PR segment", QRS complex, ST segment, ST-T segment and T wave) have been adopted for describing magnetocardiograms, which resemble electrocardiograms in many aspects, although the actual shape may vary depending on the position of the magnetic field sensor. Abbreviations like "T-Start", "$T_{start}$", "$T_{beg}$", "T-End", $T_{end}$, or the like, are used to denote the beginning or the end of a wave, interval or segment, e.g., the T wave.

The terms "QRS complex", "QRS wave" or "QRS interval" refer to a section of the heartbeat cycle representing the depolarization of the right and left ventricles of the heart and the contraction of the large ventricular muscles.

The term "PR" segment relates to the segment of the heartbeat cycle between the end of the P wave and the beginning of the QRS complex.

The term "J point", $J_p$, relates to the junction between the end of the QRS complex and the beginning of the ST segment of an electrocardiogram.

The term "ST segment" relates to a segment of an electrocardiogram representing the time interval of the cardiac cycle (heartbeat, sinus rhythm) starting after the end of the S-wave of the QRS complex, i.e., at the J point, and ending at the beginning of the T wave. The ST segment is also called the "ST interval". The ST segment represents the interval between the end of ventricular depolarization and the beginning of ventricular repolarization. The T-wave represents the repolarization of the ventricles. The terms "ST-T segment" or "ST-T interval" relate to a section comprising the ST segment and the T-wave.

The term "QT interval" refers to a section of an electrocardiogram representing the time interval between the beginning of the QRS complex and the end of the T wave. The term "QTc" refers to the duration of the QT interval corrected for the duration of the heartbeat cycle.

The term "magnetic field sensor" as used herein means a sensor being able to measure magnetic fields, e.g., a gradiometer. The term comprises a magnetic field sensor (1-axis magnetic field sensor) being designed to only measure one component of the magnetic field, e.g., the z-component, or a magnetic field sensor (2- or 3-axis magnetic field sensor) being able to measure two or all three orthogonal components (x-, y- and z-component) of the magnetic field. SQUIDs ("superconducting quantum interference devices"), e.g., having axial gradiometers as pickup coil, are preferred as sensors.

The term "measuring a component of the magnetic field at a plurality of (at least two) positions above the heart" means measuring a component, e.g., the z-component, of the magnetic field at several positions, i.e., at least at two, preferably at more than two positions, which are, preferably evenly, spaced apart from each other, e.g., by using an array of magnetic field sensors, for example 36, 52, 64 or 128 magnetic field sensors. The term "above the heart" is not to be construed as being constricted to a specific position in relation to the earth's gravitation field.

A term like "using the z-component of the magnetic field", for example in the context of the calculation of a monopolarity index, encompasses that the magnetic field data measured by 2- or 3-axis magnetic field sensors, and even by x-y-sensors only, will be converted in a manner as if the data came from an all-z axis sensor configuration, e.g., by solving for the magnetic field moment at the center of the heart, and projecting it back to a 'virtual' sensor array located above the chest.

The term "channel", if used herein, refers to the magnetic field sensors used to measure magnetic field data at different positions above a tissue or organ, e.g., the heart. The term "64-channel MCG", for example, refers to a magnetocardiography system having 64 different magnetic field sensors measuring magnetic field data.

As used herein, unless not stated otherwise or the context does not indicate otherwise, a reference to an x-axis in relation to the heart or the body of a human being corresponds to a reference to a right-to-left axis, a reference to a y-axis corresponds to a reference to a head-to-foot axis, and a reference to the z-axis corresponds to a reference to an anteroposterior axis. The term "z-component" of a magnetic field thus, for example, refers to the magnetic field component in the direction of the anteroposterior axis, i.e., an axis perpendicular to the x-y plane. The coordinates comprising a x-, y- and z-axis as explained above may also be referred to as "standard heart coordinates".

The term "subject" as used herein refers preferably to a vertebrate, further preferred to a mammal, and most preferred to a human.

The term "serum creatinine level" refers to the concentration of creatinine (2-Amino-1-methyl-5H-imidazol-4-one) in blood serum.

The terms "myocardial disorder" or "myocardial dysfunction" relate to the condition of a non-healthy, i.e., a malfunctioning and/or damaged heart muscle. In particular, the term relates to a malfunctioning and/or damaged heart muscle, e.g., as a result of cardiac ischemia.

The term "coronary artery disease", CAD, (also "chronic ischemic heart disease"; ICD-10 code I25) refers to type of heart disease in which the arteries supplying the heart muscle with blood become hardened and narrowed due to the buildup of plaque on their inner walls.

The term "acute coronary syndrome", ACS, refers to conditions, in which the blood flow to the heart muscle is suddenly stopped or severely reduced, e.g., during a myocardial infarction (see, for example, [15]).

In the application, the following numbers or indices may be used that refer to specific sections of a magnetocardiogram (using terminology originally established for electrocardiograms):

1 QRS complex
2 $QRS_{End}$ to $T_{beg}$ (ST segment)
3 $T_{beg}$ to $T_{end}$ (T wave)
4 $QRS_{end}$ to $T_{end}$ (ST-T interval)

The following abbreviations are used in the application

| | |
|---|---|
| $M_i$ | monopolarity index |
| $D_i$ | dipolarity index |
| MI | monopolarity index signal |
| $Log_{MI}$ | common logarithm of MI |
| $MI_3$ | monopolarity index signal for section 3 ($T_{beg}$-$T_{end}$) |
| $MI_4$ | monopolarity index signal for section 4 ($QRS_{end}$-$T_{end}$) |
| DI | dipolarity index signal |
| $Log_{DI}$ | common logarithm of DI |
| RMS | root mean square |
| LogRMS | common logarithm of RMS |
| $RMS_3$ | RMS value for section 3 ($T_{beg}$-$T_{end}$) |
| QTc | heart beat corrected QT interval |

The parameters MI, DI, and derivatives thereof, e.g., logMI and logDI, may also be denoted as MIG (magnetoionography) parameter. A combination of one of the parameters MI, DI or RMS with an index corresponding to the numbers given above refers to the parameter calculated for the respective cardiocycle section. For example, $MI_3$ denotes the MI values calculated for the section denoted above with the numeral 3, i.e., MI for the T wave ($T_{beg}$ to $T_{end}$).

In the method of the invention according to the first aspect of the invention magnetic field data of a magnetic field generated by the heart of a subject are acquired with a plurality, i.e., at least two, magnetic field sensors measuring at least one component, e.g., the x-, y- or z-component, or an arbitrary combination of those of a biomagnetic field for at least two different positions above the heart of the subject for a duration of at least a portion of one heartbeat cycle, the portion covering at least the QRS complex and the T wave. Further, a first and a second reference heartbeat cycle time defining the beginning and the end of an evaluation interval are selected either manually or automatically, preferably automatically, the evaluation interval covering at least the QRS complex and the T wave. The magnetic field data collected during this evaluation interval are at least partially used for the subsequent calculation of the following magnetocardiographic parameters: a reference root-mean-square value, RMS, the monopolarity index, Mi, a monopolarity index signal, MI, weighted with the reference RMS value, and an average value of the MI and RMS values.

One magnetocardiographic parameter calculated, in step c, from the magnetic field data acquired during the evaluation interval is a reference signal, the reference signal being the root-mean-square, RMS, value of the magnetic field data of the at least two magnetic field sensors for each sample acquired during the evaluation interval. The reference RMS value is the root mean square of the squares of the magnetic field data across all channels for each sample between the first and second reference cycle time.

Another magnetographic parameter calculated, in step d, from the magnetic field data acquired during the evaluation interval is the monopolarity index, Mi, of the acquired magnetic field data, the monopolarity index being the normalized sum of the magnetic field data of the at least two magnetic field sensors for each sample acquired during the evaluation interval. The monopolarity index, Mi, is calculated for each point in time a sample is taken, i.e., new magnetic field data are acquired.

From the magnetic field data acquired during the evaluation interval, or from the above monopolarity index, Mi, a monopolarity index signal, MI, of the acquired magnetic field data, is calculated, in step e, the monopolarity index signal resulting from the elementwise multiplication of the reference signal calculated in step c above with the monopolarity index calculated in step d above. Each monopolarity index value is thus weighted, for each individual sample, with the reference RMS value calculated in step c.

In a further step, step f, an average value of the MI and RMS values for at least a fraction of the evaluation interval is calculated.

At least steps c to f are preferably computer-implemented. Preferably, steps b to f are computer-implemented. For computer-processing the measured magnetic field data are digitized.

In a preferred embodiment of the method of the invention, from the average monopolarity index signal, MI, calculated in step f, an average dipolarity index signal, DI, wherein DI = 1 - MI, a logarithm of the average monopolarity index, $Log_{MI}$, and/or a logarithm of the average dipolarity index, $Log_{DI}$, is calculated, and/or, from the average RMS value calculated in step f, a logarithm of the average RMS value, $Log_{RMS}$, is calculated. These derivatives of the magnetocardiographic parameter defined above, are also useful parameters that can be used to characterize at least a portion of a heartbeat cycle in order to obtain, for example, medical relevant information for a diagnosis, prognosis, or risk prediction.

In a preferred embodiment of the method of the invention, average MI and RMS values are, independently from each other, calculated for the QRS complex, the T wave, and/or the interval from the end of the QRS complex, $QRS_{end}$, to the end of the T wave, $T_{end}$.

In a further preferred embodiment of the method of the invention, an isoline (null isoline, NI) of the magnetic field data is determined, in relation to which the magnetic field data are corrected, and wherein the isoline is determined from magnetic field data acquired during at least a portion of the heartbeat cycle where the biomagnetic strength, i.e., the strength of the biomagnetic field, is assumed to be approximately zero, preferably from magnetic field data acquired during the PR segment, i.e., the segment between the end of a P wave and the begin of the QRS complex. In this embodiment, a possible inherent sensor offset, for example, is taken into account in the calculation of reference RMS, monopolarity or dipolarity index etc., i.e., a baseline is set before calculating these parameters.

In a preferred embodiment of the method according to the first aspect of the invention the monopolarity index, Mi, of the magnetic field, representing a measure for the deviation of the magnetic field from a dipolar shape, and is calculated by the following formula (1)

$$M_i = \frac{|\sum b_i|}{\sum |b_i|} \qquad (1),$$

wherein $M_i$ is the monopolarity index, *i* the sensor index and $b_i$ the magnetic field gradient measured by the i-th magnetic field sensor.

In this embodiment, the at least one component of the biomagnetic field is preferably measured at at least two positions above the heart of the subject using at least two magnetic field sensors. Each sensor of the at least two of magnetic field sensors is arranged at one of the positions of the at least two positions above the heart of the subject. The monopolarity is calculated based on the magnetic field data of the at least two magnetic field sensors. As an example, in case of e.g., 64 magnetic field sensors ($i$ = 1,...,64) measuring e.g., the z component of the magnetic field, the data measured by each of the 64 sensors is summed up, and the absolute value of this sum is then divided by the sum of the absolute values of each of the sensors. The processing of the magnetic field data of the at least two magnetic field sensors for calculating the monopolarity is computer-implemented. The magnetic field data are thus digitized and processed by a computer.

The above formula provides a monopolarity index (or monopolarity score) lying between 0 (zero, no monopolarity) and 1 (one, complete monopolarity). A perfect dipolar magnetic field would thus have a zero score, whereas any magnetic field deviating from a dipolar shape and having a more monopolar shape would have a score greater than zero.

It is preferred that, in the method according to the first aspect of the invention, the at least one component of the biomagnetic field is measured at more than two positions above the heart of the subject using more than two magnetic field sensors, each sensor being arranged at one of the positions above the heart of the subject. As an example, the at least one component of the biomagnetic field can be measured at 36, 52, 64, or 128 positions above the heart of the subject using 36, 52, 64, or 128 magnetic field sensors (channels).

In a preferred embodiment of the method according to the first aspect of the invention, an averaged dispersion of the T wave is calculated using the magnetic field data acquired in step a, the averaged dispersion of the T wave being determined by detecting the time-point of the maximum value (maximum amplitude) of the T wave ($T_{max}$) and calculating the standard deviation of the magnetic field data measured by the at least two magnetic field sensors at that time-point.

In a particular preferred embodiment of the method of the invention according to the first aspect of the invention, the magnetocardiographic parameters are at least determined for the T wave, further preferred at least for the QT interval, i.e., the interval between the start of the QRS complex and the end of the T wave.

As mentioned above, the method of the invention according to the first aspect can, for example, be particularly useful in the context of the evaluation of myocardial dysfunctions, e.g., for the prediction of a risk for sudden cardiac death, SCD, in a subject.

The measurement of the magnetic field component may be performed under resting conditions, e.g., under conditions where the subject rests on a bed or suchlike, or under stress conditions, i.e., under conditions of physical stress, e.g., during exercises performed with an ergometer or suchlike. It is preferred to perform the measurements on subjects at rest, further preferred first at rest and during recovery after an exercise period.

Although it is possible to take any component, i.e., the x-, y- or z-component, of the magnetic field for the determination of its monopolarity, it is preferred to use the z-component for this purpose. The z-component is the component usually measured by magnetocardiography. Using the z-component does not mean that only one-axis magnet field sensors, e.g., gradiometers measuring only the z-component, can be implemented.

In the method according to the first aspect of the invention, at least the calculation steps c to f are computer-implemented. The method of the invention is, however, preferably completely automated, in that the magnetic field data acquired in step a are automatically passed in digitized form to step b for automatically selecting a first and second reference heartbeat cycle time, i.e., for automatically defining an evaluation interval, and to the calculation steps c to f. A desired evaluation interval, e.g., the QT interval, can also be predefined, and the method of the invention is configured to automatically determine, from the measured magnetic field data, the beginning and the end of the desired interval, i.e., the first and second reference heartbeat cycle times. The digitized data of step a are then computed in a suitable manner, e.g., by means of a computer program running, for example, on a personal computer or another programmable apparatus, in order to determine, using the magnetic field data measured by the at least one magnetic field sensor, the magnetocardiographic parameter of the invention.

In a second aspect the invention relates to a method for predicting a risk for sudden cardiac death in a subject, in particular a subject having or being suspected to have coronary artery disease, CAD, further in particular acute coronary syndrome, ACS, the method using the following parameters:

a) the serum creatinine level in the blood of the subject;
b) QTc, defined as the heart rate corrected duration between the start of the QRS complex, $QRS_{beg}$, and the end of the T wave, $T_{end}$; and
c) at least one magnetocardiographic parameter obtained using the method according to the first aspect of the invention, described above, the at least one magnetocardiographic parameter being selected from the group consisting of the following magnetocardiographic parameters:

i. $MI_3$ = average monopolarity index signal of T wave,
ii. $Log_{MI3}$ = logarithm of average monopolarity of T wave,
iii. $MI_4$ = average monopolarity of the interval from the end of the QRS complex, $QRS_{end}$, to the end of the T wave, $T_{end}$,
iv. averaged dispersion of T wave, calculated from the standard deviation of the magnetic field data at the maximum amplitude of the T wave, $T_{max}$.

v. $RMS_3$ = average of root mean square values for the T wave, and

vi. $Log_{RMS3}$ = logarithm of average of root mean square values for the T wave.

The parameters $MI_3$ and $Log_{MI3}$ may also be denoted as "repolarization reserve". The parameter iv, i.e., the averaged dispersion of the T wave, may also be abbreviated with the term "T dispersion".

As an additional parameter, that can also be determined by magnetocardiography and thus also with the method of the invention, the heart rate can be used.

The method of the invention has surprisingly been shown to be able to predict a risk for sudden cardiac death, SCD, with unprecedented reliability. Increased serum creatinine level, prolonged QTc and, for example, and a low monopolarity index signal, have been shown to highly correlate with the risk to encounter sudden cardiac death. Based on the method of the invention, it is possible to define an SCD risk score to assess a risk of a subject to encounter a sudden cardiac death in the future.

In a particularly preferred embodiment of the method of the invention according to the second aspect of the invention, the at least one magnetocardiographic parameter is selected from the parameters i.-iv. above, particularly preferred form the parameters i. to iii. above, i.e., from the parameters $MI_3$, $Log_{MI3}$, and $MI_4$. $MI_3$ and $Log_{MI3}$ provide information on the repolarization current IKr + IKs during the T wave, and low values for these parameters are indicative of a depression of the repolarization current IKr+IKs. The parameter $MI_4$ relates to the monopolarity index signal of the ST-T interval, also covering the T wave. These parameters can, for example, be advantageously be used to define an SCD risk score as mentioned above.

In a preferred embodiment of the method of the invention according to the second aspect of the invention, QTc is automatically calculated from magnetic field data acquired in step a of the method according to the first aspect of the invention. In this embodiment, magnetic field data of at least one full heartbeat cycle are acquired, from which the duration of the heartbeat is determined in order to correct the QT interval for the duration of the heartbeat. It is also possible, of course, to collect data over a series of consecutive heartbeat cycles, from which the heart rate and the average duration of a heartbeat cycle may be calculated.

In a third aspect, the invention also relates to a magnetocardiographic system, comprising a plurality of, i.e., at least two, magnetic field sensors being configured to measure a component of the magnetic field generated by a heart of a subject, and a calculation unit being configured to carry out the method of the invention according to the first aspect of the invention, as described above.

The calculation unit may, for example, be an integrated circuit, a Personal Computer (PC) or other programmable apparatus, or part thereof, being configured to perform a calculation. In a preferred embodiment of the third aspect of the invention, the magnetocardiographic system comprises a Personal Computer running a program being configured to determine the magnetocardiographic parameters, e.g., the monopolarity index, of the magnetic field. The magnetocardiographic system of the invention may thus be composed of a magnetocardiograph being designed and configured to measure, with a plurality of, i.e., at least two magnetic field sensors, e.g., SQUID sensors coupled to axial or planar gradiometers being configured to measure the z-component of a magnetic field of the heart of a subject, and a Personal Computer connected to the magnetocardiograph and configured to acquire and process the magnetic field data and to determine, from the magnetic field data, magnetocardiographic parameters such as Mi, Di, MI, DI, logMI, logDI, RMS and logRMS, as described in more detail above.

In a preferred embodiment of the magnetocardiographic system of the invention, the calculation unit is configured to calculate a monopolarity index, Mi, according to the following formula (1)

$$M_i = \frac{|\sum b_i|}{\sum |b_i|} \qquad (1),$$

wherein $M_i$ is the monopolarity index, *i* the sensor index and $b_i$ the magnetic field gradient measured by the i-th magnetic field sensor.

Preferably, the calculation unit of the magnetocardiographic system of the invention is configured to determine the monopolarity index, Mi, of specific sections of a heartbeat, for example the T wave and/or the ST-T interval, as described above in more detail, e.g., $MI_3$ (the average monopolarity index signal of the T wave) and/or $MI_4$ (the average monopolarity index of the ST-T interval) and/or derivatives thereof, e.g., $LogMI_3$. Further preferred, the calculation unit is configured to calculate the RMS value, as described above, for specific sections of the heartbeat, for example $RMS_3$ (average of root mean square values for the T wave), and derivatives thereof, e.g., $Log_{RMS3}$ (logarithm of average of root mean square values for the T wave).

Preferably, the plurality of, i.e., at least two and preferably more, magnetic field sensors of the magnetocardiographic system of the invention is configured to measure the z-component of the magnetic field. The magnetic field sensors may, for example, be planar gradiometers or axial first- or second-order gradiometers coupled to a SQUID and oriented in a manner to be able to measure the z-component of the magnetic field, i.e., the component perpendicular to the x-y plane (frontal plane) according to standard heart coordinates. Such sensors are known to the skilled person.

In a fourth aspect, the invention relates to a data carrier, i.e., a computer readable medium, comprising a computer program for carrying out the method according to the first aspect of the invention.

In the following the invention is further described for illustration purposes only by way of the attached figures.

Figure 1. Schematic drawing of a simplified magnetocardiogram.

Figure 2. Scatter plot of QTc vs Repolarization reserve $Log_{MI3}$ clearly separates the survivors and the cardiac deaths.

Figure 3. Cumulative survival functions of Kaplan Meier analysis using the three significant parameters from Cox regression (table 2) and predicted group memberships from table 3.

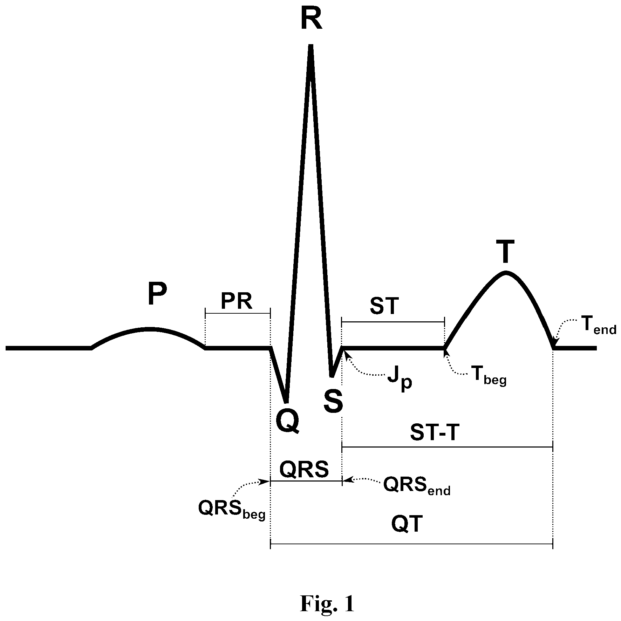

Figure 1 schematically shows an idealized magnetocardiogram of a heartbeat cycle (cardiac cycle) of a normal healthy subject. It is to be noted that only the data of one sensor at a specific position is depicted. Magnetocardiograms differ depending on the position of the sensor in relation to the magnetic field. In Figure 1, P denotes the P wave, Q the Q wave, R the R wave, S the S wave, and QRS the QRS complex. $QRS_{beg}$ and $QRS_{end}$ denote the beginning and end of the QRS complex, $T_{beg}$ and $T_{end}$ the beginning and end of the T wave. The ST segment, the ST-T segment and the J point are also marked. QT denotes the QT interval, i.e., the interval from $QRS_{beg}$ to $T_{end}$.

Example

Prediction of cardiac death in patients with acute coronary syndrome

Acute coronary syndrome (ACS) refers to a number of cardiac conditions associated with an unmediated reduction in blood flow to the heart [15]. One such condition may be a heart attack, in which there is pathological change in the heart tissue. Even though ACS does not cause cell death, the resulting reduced blood flow changes the way the heart functions and leads to a high risk of myocardial infarction or sudden cardiac death. ACS is a medical emergency that requires rapid diagnosis and treatment. The following describes analysis of ACS patients treated at Yonsei University Hospital (Seoul, South Korea). It should be noted that the mortality of the patients in this study is low in comparison with other investigations [16].

Methods

Patients

Magnetocardiograms (MCGs) were recorded from 245 patients before coronary angiography between November 2008 and July 2009. The measurements of 191 patients with ACS were retrospectively analyzed. All patients were recruited in 2009 at Yonsei University Hospital (Seoul, South Korea) and were followed up until 2022. The study was approved by the local ethics committee and informed consent was obtained from each subject.

Magnetocardiography (MCG)

MCG can measure tangential currents that occur at the boundary between damaged and healthy tissue, which the ECG cannot detect. In addition, MCG is able to measure electrically silent phenomena - intracellular ionic currents as well as vortex currents.

The measurement system contained 64 channels evenly distributed inside the cryogenic Dewar with a diameter of 200 mm. Each channel was a first-order planar gradiometer connected to a DC LT-SQUID that measures the tangential component of the cardiomagnetic field. The average noise level of the entire system in a magnetically shielded room is 10 fT in unit band. The system simultaneously registers ECG in 12 leads. The recording time at rest was 2 min with an acquisition rate of 500 Hz. Raw signals were then averaged and baseline corrected.

MCG parameters were calculated for the following cardiocycle sections:

1 - QRS (QRS complex)
2 - $QRS_{end}$ - $T_{beg}$ (ST segment),
3 - $T_{beg}$ - $T_{end}$ (T wave),
4 - $QRS_{end}$ - $T_{end}$ (ST-T interval).

The combination of the relevant MCG parameter (e.g., RMS or MI) with the number of the above-mentioned sections gives the parameter used. For example, $RMS_1$ means the average root mean square of all 64 channels during QRS. The following table 1 provides an overview over parameters used:

Table 1: Overview of MCG parameter

|  | Parameter [unit] | Explanation |
|---|---|---|
| 1 | $MI_1$ [pT] | Average monopolarity index signal of QRS complex |
| 2 | $DI_1$ [pT] | Average dipolarity index signal of QRS complex |
| 3 | $MI_2$ [pT] | Average monopolarity index signal of $QRS_{end}$ - $T_{beg}$ |
| 4 | $DI_2$ [pT] | Average dipolarity index signal of $QRS_{end}$ - $T_{beg}$ |
| 5 | $MI_3$ [pT] | Average monopolarity index signal of $T_{beg}$ - $T_{end}$ |
| 6 | $DI_3$ [pT] | Average dipolarity index signal of $T_{beg}$ - $T_{end}$ |
| 7 | $MI_4$ [pT] | Average monopolarity index signal of $QRS_{end}$ - $T_{end}$ |
| 8 | $DI_4$ [pT] | Average dipolarity index signal of $QRS_{end}$ - $T_{end}$ |
| 9 | $Log_{MI3}$ | Logarithm of $MI_3$ |
| 10 | Ca.uptake velocity [pT/sec] | Maximum negative slope of the dipolarity index signal between $T_{max}$ and $T_{end}$ |
| 11 | $RMS_1$ [pT] | Average of root mean square for QRS complex |
| 12 | $RMS_2$ [pT] | Average of root mean square for $QRS_{end}$ - $T_{beg}$ |
| 13 | $RMS_3$ [pT] | Average of root mean square for $T_{beg}$ - $T_{end}$ |
| 14 | $RMS_4$ [pT] | Average of root mean square for $QRS_{end}$ - $T_{end}$ |
| 15 | $Log_{RMS3}$ | Logarithm of $RMS_3$ |

The parameters 1-10 are also denoted here as magnetoionography (MIG) parameters (see table 4 below). The parameters 11-15 are denoted as MCG parameters in the narrow sense. The MIG parameters MI and DI stand, for example, for "monopolarity index signal" and "dipolarity index signal", respectively. MI and DI represent the "monopolarity index", Mi, or "dipolarity index", Di, weighted by RMS in the respective intervals.

Statistical Analysis

Stepwise Cox regression analysis was performed in SPSS (v.27) and allows to adjust for several variables. A P-value below 0.05 was considered significant. Linear discriminant analysis (LDA) [17] was used to obtain a classification rule to separate the survivors from the deceased. The robustness of the resulting classification was checked by means of a leave one out cross-validation which has been proven to minimize bias and mean squared error in an LDA [18]. The nonparametric Mann-Whitney U test was deployed to assess if there is a significant difference in the clinical characteristics between the two groups [19]. Kaplan-Meier analysis was used to assess the relation between different predicted group memberships and survival (years) [20].

Results

191 patients with acute coronary syndrome (age 62.5±10.5 years; 77 men [59.7%)], 114 women [40.3%]) were recruited between November 2008 and July 2009 at Severance Hospital (Yonsei University, Seoul, South Korea). Patients were admitted to the hospital with chest pain without ST segment elevation in electrocardiography. The ACS diagnosis was based on typical chest pain, cardiac enzyme (plasma creatine kinase (CK) cardiac isoenzyme level) and echocardiographic findings. Coronary angiography was performed and left ventricular ejection fraction (LVEF) with echocardiography was obtained in every patient. All patients were followed up until 2022. During half of the follow up period (6.5 years) 11 patients died. Clinical characteristics of the 6.5 years survivors vs. the deceased are given in table 2.

Table 2: Clinical characteristics of 180 ACS patients, which survived the 6.5 years follow up vs. 11 deceased patients. ns = not significant.

| Parameter | Survivors (n=180) | Cardiac Death (n=11) | P-value |
|---|---|---|---|
| Age | 62.0 ± 11.1 | 64.4 ± 10.2 | ns |
| Gender | 110 (f) | 4 (f) | ns |
| BMI | 24.8 ± 3.2 | 22.2 ± 4.7 | ns |
| Smoking | 48 | 2 | ns |
| Dyslipidemia | 40 | 4 | ns |
| Diabetes | 54 | 5 | ns |
| MI | 6 | 2 | ns |
| Revascularization | 18 | 2 | ns |
| Creatinine (mg/dL) | 1.0 ± 0.3 | 1.3 ± 0.6 | p<0.01 |

Results for the MCG parameters (in the narrow sense) and MIG parameters used are given in tables 3 and 4 below.

Table 3: Results of heart rate and the MCG parameters (in the narrow sense) in patients, which survived the 6.5 years follow up vs. the deceased patients ($Log_{RMS3}$ is the logarithm of $RMS_3$). n.s. = not significant.

| | Survivors (n=180) | Cardiac Death (n=11) | P-value |
|---|---|---|---|
| Heart Rate [bpm] | 64.0±11.5 | 75.7 ± 17.7 | 0.014 |
| QRS [ms] | 96.2±19.6 | 99.6 ± 11.7 | ns |
| T-Dispersion [ms] | 9.3 ± 2.5 | 11.3 ±2.5 | 0.014 |
| QTc [ms] | 391.2 ± 40.0 | 445.2 ±55.8 | 0.001 |
| $RMS_1$ [pT] | 3.4 ± 1.8 | 2.7 ± 1.2 | ns |
| $RMS_2$ [pT] | 0.72 ± 0.67 | 0.51 ± 0.2 | ns |
| $RMS_3$ [pT] | 1.3 ± 0.9 | 0.84 ± 0.3 | 0.037 |
| $RMS_4$ [pT] | 1.02 ± 0.76 | 0.70 ± 0.2 | ns |
| $LOg_RMS3$ | 0.05 ± 0.25 | -0.11 ± 0.2 | 0.037 |

Table 4: Results of the MIG parameters in patients, which survived the 6.5 years follow up vs. the deceased patients ($Log_{MI3}$ is the logarithm of $MI_3$).

| | Survivors (n=180) | Cardiac Death (n=11) | P-value |
|---|---|---|---|
| $MI_1$ [pT] | 1.76 ± 1.1 | 1.56 ± 0.91 | ns |
| $DI_1$ [pT] | 1.7 ± 1.2 | 1.14 ± 0.46 | ns |
| $MI_2$ [pT] | 0.37 ± 0.41 | 0.28 ± 0.17 | ns |
| $DI_2$ [pT] | 0.34 ± 0.4 | 0.23 ± 0.17 | ns |
| $MI_3$ [pT] | 0.53 ± 0.56 | 0.17 ± 0.11 | 0.001 |
| $DI_3$ [pT] | 0.8 ± 0.66 | 0.67 ± 0.24 | ns |
| $MI_4$ [pT] | 0.45 ± 0.44 | 0.23 ± 0.12 | 0.014 |
| $DI_4$ [pT] | 0.57 ± 0.53 | 0.46 ±0.2 | ns |
| $Log_{MI3}$ | -0.43 ± 0.37 | -0.84 ± 0.27 | 0.001 |
| Ca.uptake velocity [pT/sec] | (-28.2) ± 24.2 | (-20.1) ± 7.7 | ns |

Out of all included 9 clinical, 8 magnetocardiographical (cf. Tab. 3) and 9 newly introduced magnetoionographical (cf. Tab. 4; MIG) parameters that were tested in this study three parameters revealed as outstanding in predicting death: QTc prolongation, depression of repolarization current IKr + IKs ($MI_3$ or $Log_{MI3}$) and serum creatinine (all significant in Cox regression, table 5, $p < 0.05$).

Table 5: Significant parameters in the COX regression model.

|  | B | SE | Wald | Df | Sig. | Exp(B) |
|---|---|---|---|---|---|---|
| Smoking | .065 | .957 | .005 | 1 | .9459 | 0.937 |
| Dyslipidemia | .809 | .700 | 1.334 | 1 | .2480 | 0.445 |
| DM | .053 | .709 | .006 | 1 | .9404 | 0.948 |
| Cr | 1.265 | .613 | 4.263 | 1 | .0390 | 3.543 |
| QTc | .023 | .007 | 12.800 | 1 | .0003 | 1.024 |
| $LOG_{MI3}$ | -3.757 | 1.262 | 8.868 | 1 | .0029 | .023 |
| Age | .014 | .041 | .119 | 1 | .7296 | 1.014 |
| Sex | -.503 | .829 | .368 | 1 | .5442 | 1.654 |

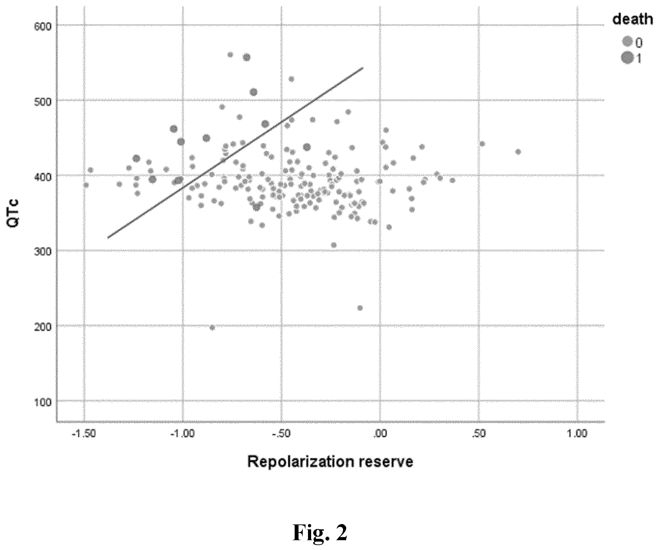

The best two parameters QTc prolongation and the repolarization reserve $Log_{MI3}$ demonstrate the classification potential in Figure 2. Almost all deceased (large circles) have higher QTc values and a decreased repolarization reserve $Log_{MI3}$. The negative predictive value is about 99 % (cf. values below).

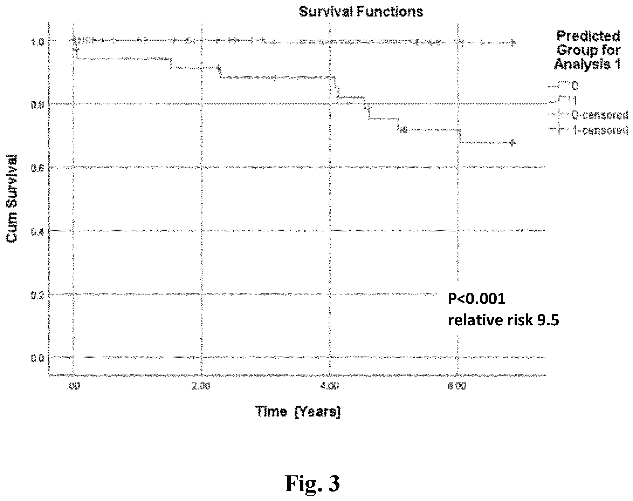

QTc, repolarization reserve and serum creatinine clearly predicted cardiac death over the 6.5 years duration (Table 6, sensitivity 90.9 %, specificity 85.6 %, negative predictive value 99.4 %). Cardiac death risk was more than 9-fold higher in patients with low repolarization reserve and QTc prolongation in comparison to the remaining ACS patients (Fig. 3, $p < 0.001$). The non-parametric Kaplan-Meier statistics estimated significantly lower survival functions from their lifetime data ($p < 0.001$).

Table 6: Classification results[a,c] of linear discriminant function analysis using QTc, $Log_{MI3}$ and Cr (serum creatinine) for the separation of the survivors (group 0) and the cardiac death group (1). Cross validated sensitivity is 90.9% and specificity 85.6%.

|  |  | Death | Predicted Group Membership | | Total |
|---|---|---|---|---|---|
|  |  |  | 0 | 1 |  |
| Original | Count | 0 | 155 | 25 | 180 |
|  |  | 1 | 1 | 10 | 11 |
|  | % | 0 | 86.1 | 13.9 | 100.0 |
|  |  | 1 | 9.1 | 90.9 | 100.0 |
| Cross-validated[b] | Count | 0 | 154 | 26 | 180 |
|  |  | 1 | 1 | 10 | 11 |
|  | % | 0 | 85.6 | 14.4 | 100.0 |
|  |  | 1 | 9.1 | 90.9 | 100.0 |

a. 86.4% of original grouped cases correctly classified.
b. Cross validation is done only for those cases in the analysis. In cross validation, each case is classified by the functions derived from all cases other than that case.
c. 85.9% of cross-validated grouped cases correctly classified.

In a validation statistics all 9 magnetoionographical parameters (see table 4 above) were left out of consideration. From the residual set of parameters again QTc and Cr were automatically selected, instead of $log_{MI3}$ the $log_{RMS3}$ was included. Discriminant function analysis led to a cross-validated sensitivity of 86.7%, a specificity of 81.1% and a negative predictive value of 98.7% (the percentage of correctly classified persons is 86.4 % and is similar to the previous discrimination).

The study described above investigated the potential of magnetocardiography at rest to predict mortality in patients with ACS. The main finding was that, for example, QTc prolongation, increased serum creatinine and a decreased repolarization reserve ($Log_{RMS3}$) from MCG at rest are very sensitive and specific to predict mid-to-long-term mortality of ACS patients. The sensitivity of 90.9 % for cardiac death, its specificity of 85.6 % as well as its outstanding negative predictive value of 99.4 % offers new possibilities in clinical diagnostics. The findings allow new prevention strategies and herald MCG techniques, in particular when configured to carry out the method of the invention according to the first aspect, as complementary risk stratification tools - not only for ACS patients.

References

[1] Macfarlane PW, et al., "The Early Repolarization Pattern: A Consensus Paper", Journal of the American College of Cardiology 66, No. 4 (28. July 2015): 470-77, https://doi.org/10.1016/j .j acc.2015.05.033.

[2] Erling Falk, Prediman K. Shah, und Valentin Fuster, "Coronary Plaque Disruption", Circulation 92, Nr. 3 (1. August 1995): 657-71, https://doi.org/10.1161/01.CIR.92.3.657.

[3] Ezra A. Amsterdam u. a., "2014 AHA/ACC Guideline for the Management of Patients With Non-ST-Elevation Acute Coronary Syndromes", Journal of the American College of Cardiology 64, Nr. 24 (23. December 2014): e139-228, https://doi.org/10.1016/j .j acc.2014.09.017.

[4] Milad Matta u. a., "Stress Testing and Noninvasive Coronary Imaging: What's the Best Test for My Patient?", Cleveland Clinic Journal of Medicine 88, Nr. 9 (1. September 2021): 502-15, https://doi.org/10.3949/ccjm.88a.20068.

[5] Taylor Dowsley et al., "The Role of Noninvasive Imaging in Coronary Artery Disease Detection, Prognosis, and Clinical Decision Making", Canadian Journal of Cardiology 29, No. 3 (1. March 2013): 285-96, https://doi. org/10.1016/j.cjca.2012.10.022.

[6] Robert Detrano, Renato Gianrossi, und Victor Froelicher, "The Diagnostic Accuracy of the Exercise Electrocardiogram: A Meta-Analysis of 22 Years of Research", Progress in Cardiovascular Diseases 32, Nr. 3 (1. November 1989): 173-206, https://doi.org/10.1016/0033-0620(89)90025-X

[7] Obeyesekere MN, Klein GJ, Nattel S, Leong-Sit P, Gula LJ, Skanes AC, Yee R, Krahn AD. A clinical approach to early repolarization. Circulation. 2013 Apr 16;127(15):1620-9. doi: 10.1161/CIRCULATIONAHA.112.143149. PMID: 23588960.

[8] Hayashi, M., Shimizu, W., Albert, C.M., 2015. The Spectrum of Epidemiology Underlying Sudden Cardiac Death. Circ. Res. 116, 1887-1906, doi: 10.1161/CIRCRESAHA116.304521

[9] Gerber, Y., Jacobsen, S.J., Frye, R.L., Weston, S.A., Killian, J.M., Roger, V.L., 2006. Secular trends in deaths from cardiovascular diseases: a 25-year community study, Circulation 113, 2285-2292, doi: 10.1161/CIRCULATIONAHA.105.590463

[10] Aziz, E.F., Javed, F., Pratap, B., Herzog, E., 2010, Strategies for the prevention and treatment of sudden cardiac death, Open Access Emerg. Med. OAEM 2010, 99-114. doi: 10.2147/OAEM.S6869.

[11] Burke, A.P., Farb, A., Malcom, G.T., Liang, Y.H., Smialek, J., Virmani, R., 1997. Coronary risk factors and plaque morphology in men with coronary disease who died suddenly. N. Engl. J. Med. 336, 1276-1282, doi: 10.1056/NEJM199705013361802

[12] Friedlander, Y., Siscovick, D.S., Weinmann, S., Austin, M.A., Psaty, B.M., Lemaitre, R.N., Arbogast, P., Raghunathan, T.E., Cobb, L.A., 1998. Family history as a risk factor for primary cardiac arrest. Circulation 97, 155-160, doi: 10.1161/01.cir.97.2.155

[13] Røe, A.T., Frisk, M., Louch, W.E., 2015. Targeting cardiomyocyte Ca2+ homeostasis in heart failure. Curr. Pharm. Des. 21, 431-448, doi: 10.2174/1381612821041411204124129

[14] Bers, D.M., 2006. Altered cardiac myocyte Ca regulation in heart failure. Physiol. Bethesda Md 21, 380-387, doi: 10.1152/physiol.00019.2006

[15] Amsterdam, E.A., Wenger, N.K., Brindis, R.G., Casey, D.E., Ganiats, T.G., Holmes, D.R., Jaffe, A.S., Jneid, H., Kelly, R.F., Kontos, M.C., Levine, G.N., Liebson, P.R., Mukherjee, D., Peterson, E.D., Sabatine, M.S., Smalling, R.W., Zieman, S.J., 2014. 2014 AHA/ACC Guideline for the Management of Patients With Non-ST-Elevation Acute Coronary Syndromes. Circulation 130, e344-e426, doi:10.1161/CIR.0000000000000134

[16] Kolansky, DM, 2009, Acute coronary syndromes: morbidity, mortality, and pharmacoeconomic burden. Am J Manag Care. 2009 Mar;15(2 Suppl):S36-41. PMID: 19355807.

[17] Fisher, R.A., 1936. The Use of Multiple Measurements in Taxonomic Problems. Ann. Eugen. 7, 179-188. Doi:/10.1111/j.1469-1809.1936.tb02137.x

[18] Molinaro, A.M., Simon, R., Pfeiffer, R.M., 2005. Prediction error estimation: a comparison of resampling methods. Bioinformatics 21, 3301-3307. doi: 10.1093/bioinformatics/bti499

[19] Fay, M.P., Proschan, M.A., 2010. Wilcoxon-Mann-Whitney or t-test? On assumptions for hypothesis tests and multiple interpretations of decision rules. Stat. Surv. 4, 1-39. doi: 10.1214/09-SS051

[20] Bewick, V., Cheek, L., Ball, J., 2004. Statistics review 12: Survival analysis. Crit. Care 8, 389-394. Doi: 10.1186/cc2955

[21] Vinogradova, T.M.; Zhou, Y.Y.; Maltsev, V.; Lyashkov, A.; Stern, M.; Lakatta, E.G. Rhythmic ryanodine receptor Ca2+ releases during diastolic depolarization of sinoatrial pacemaker cells do not require membrane depolari-zation. Circ. Res. 2004, 94, 802-809.

**Claims**

1. A method for the magnetocardiographic characterization of at least a portion of a heartbeat cycle of a heart of a subject, the method comprising:

   a) acquiring magnetic field data with at least two magnetic field sensors measuring at least one component of a biomagnetic field at at least two different positions above the heart of the subject for a duration of at least a portion of one heartbeat cycle, the portion covering at least the QRS complex and the T wave;
   b) selecting a first and a second reference heartbeat cycle time defining the beginning and the end of an evaluation interval, the evaluation interval covering at least the QRS complex and the T wave;
   c) calculating, from the magnetic field data acquired during the evaluation interval, a reference signal, the reference signal being the root-mean-square, RMS, value of the magnetic field data of the at least two magnetic field sensors calculated for each sample acquired during the evaluation interval;
   d) calculating, from the magnetic field data acquired during the evaluation interval, a monopolarity index, Mi, of the acquired magnetic field data, the monopolarity index being the normalized sum of the magnetic field data of the at least two magnetic field sensors for each sample acquired during the evaluation interval;
   e) calculating, from the magnetic field data acquired during the evaluation interval, or from the monopolarity index, Mi, calculated in step d, a monopolarity index signal, MI, of the acquired magnetic field data, the monopolarity index signal resulting from the elementwise multiplication of the reference signal calculated in step c above with the monopolarity index calculated in step d above; and
   f) calculating an average value of the MI and RMS values for at least a fraction of the evaluation interval;

   wherein steps c to f are computer-implemented.

2. The method of claim 1, wherein, from the average monopolarity index signal, MI, calculated in step f, an average dipolarity index signal, DI, wherein DI = 1 - MI, a logarithm of the average monopolarity index, $Log_{MI}$, and/or a logarithm of the average dipolarity index, $Log_{DI}$, is calculated, and/or, from the average RMS value calculated in step f, a logarithm of the average RMS value, $Log_{RMS}$, is calculated.

3. The method of one of the preceding claims, wherein in step f, average MI and RMS values are, independently from each other, calculated for the QRS complex, the T wave, and/or the interval from the end of the QRS complex, $QRS_{end}$, to the end of the T wave, $T_{end}$.

4. The method of one of the preceding claims, wherein an isoline of the magnetic field data is determined, in relation to which the magnetic field data are corrected, and wherein the isoline is determined from magnetic field data acquired during at least a portion of the heartbeat cycle where the strength of the biomagnetic field is assumed to be approximately zero, preferably from magnetic field data acquired during the segment between the end of a P wave and the begin of the QRS complex.

5. The method of one of the preceding claims, wherein the monopolarity index of the magnetic field is calculated using the following formula (1)

$$M_i = \frac{|\sum b_i|}{\sum |b_i|} \qquad (1),$$

   wherein $M_i$ is the monopolarity index, $i$ the sensor index and $b_i$ the magnetic field gradient measured by the i-th magnetic field sensor.

6. The method of one of the preceding claims, wherein an averaged dispersion of the T wave is calculated using the magnetic field data acquired in step a, the averaged dispersion of the T wave being calculated as the standard deviation of the magnetic field data at the maximum amplitude of the T wave.

7. The method according to one of the preceding claims, wherein, in step a, magnetic field data are acquired with three or more magnetic field sensors, preferably with 36, 52, 64 or more magnetic field sensors, measuring at least one component of a biomagnetic field at three or more different positions above the heart of the subject.

8. The method according to one of the preceding claims, wherein the component of the magnetic field is the z-component of the magnetic field.

9. The method according to one of the preceding claims, wherein steps b to f are computer-implemented.

10. The method according to one of the preceding claims, wherein the subject is a mammal, preferably a human.

11. A method for predicting a risk for sudden cardiac death in a subject having or being suspected to have coronary artery disease, CAD, in particular acute coronary syndrome, ACS, the method using the following parameters:

a) the serum creatinine level in the blood of the subject;
b) QTc, defined as the heart rate corrected duration between the start of the QRS complex, $QRS_{beg}$, and the end of the T wave, $T_{end}$; and
c) at least one magnetocardiographic parameter obtained using the method of one of claims 1 to 10, the at least one magnetocardiographic parameter being selected from the group consisting of the following magnetocardiographic parameters:

i. $MI_3$ = average monopolarity index signal of T wave,
ii. $Log_{MI3}$ = logarithm of average monopolarity of T wave,
iii. $MI_4$ = average monopolarity of the interval from the end of the QRS complex, $QRS_{end}$, to the end of the T wave, $T_{end}$,
iv. averaged dispersion of T wave, being calculated as the standard deviation of the magnetic field data at the maximum amplitude of the T wave,
v. $RMS_3$ = average of root mean square values for the T wave, and
vi. $Log_{RMS3}$ = logarithm of average of root mean square values for the T wave.

12. The method of claim 11, wherein the at least one magnetocardiographic parameter is selected from the parameters i. to iii. above, preferably selected from the parameters i. or ii.

13. The method of claim 11 or 12, wherein QTc is automatically calculated from magnetic field data acquired in step a of the method according to one of claims 1 to 10.

14. Magnetocardiographic system, comprising at least two magnetic field sensors being configured to measure a component of the magnetic field generated by a heart of a subject, and a calculation unit being configured to carry out the method of one of claims 1 to 10.

15. Magnetocardiographic system according to claim 14, wherein the at least two magnetic field sensors are configured to measure the z-component of the magnetic field.

16. Magnetocardiographic system according to one of claims 14 to 15, comprising a computer being or comprising a calculation unit performing the calculations in the method of one of claims 1 to 10.

17. Data carrier comprising a computer program for carrying out steps c to f, preferably steps b to f, of the method of one of claims 1 to 10.

**Fig. 1**

**Fig. 2**

**Survival Functions**

**Fig. 3**

# EP 4 491 119 A1

Europäisches
Patentamt

European
Patent Office

Office européen
des brevets

# EUROPEAN SEARCH REPORT

Application Number

EP 23 18 5262

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 3 308 703 A1 (BIOMAGNETIK PARK GMBH [DE]) 18 April 2018 (2018-04-18) * paragraph [0001] – paragraph [0039]; figures 1-5 * | 1-17 | INV. A61B5/243 A61B5/318 A61B5/349 A61B5/366 |
| Y | US 2008/194978 A1 (BEKER AMIR [IL] ET AL) 14 August 2008 (2008-08-14) * abstract; paragraph [0005] – paragraph [0258]; claim 62; figures 1-15 * | 1-17 | A61B5/355 A61B5/00 |
| A | US 2002/143265 A1 (ACKERMAN MICHAEL J [US] ET AL) 3 October 2002 (2002-10-03) * paragraph [0024] – paragraph [0057]; figures 1-6 * | 1-17 | |
| A | US 2009/088655 A1 (VAJDIC BRANISLAV [US] ET AL) 2 April 2009 (2009-04-02) * abstract; figures 1-11 * | 1-17 | |
| A | US 2007/203418 A1 (STARC VITO [SI]) 30 August 2007 (2007-08-30) * paragraph [0027] – paragraph [0133]; figures 1-14 * | 1-17 | TECHNICAL FIELDS SEARCHED (IPC) A61B G16H |

The present search report has been drawn up for all claims

| Place of search | Date of completion of the search | Examiner |
|---|---|---|
| The Hague | 5 October 2023 | Munteh, Louis |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 18 5262

This annex lists the patent family members relating to the patent documents cited in the above-mentioned European search report.
The members are as contained in the European Patent Office EDP file on
The European Patent Office is in no way liable for these particulars which are merely given for the purpose of information.

05-10-2023

| Patent document cited in search report | | Publication date | Patent family member(s) | | Publication date |
|---|---|---|---|---|---|
| EP 3308703 | A1 | 18-04-2018 | EP | 3308703 A1 | 18-04-2018 |
| | | | WO | 2018069287 A1 | 19-04-2018 |
| US 2008194978 | A1 | 14-08-2008 | AU | 2005237329 A1 | 10-11-2005 |
| | | | CA | 2565192 A1 | 10-11-2005 |
| | | | CN | 101014283 A | 08-08-2007 |
| | | | EP | 1746932 A2 | 31-01-2007 |
| | | | JP | 2007535392 A | 06-12-2007 |
| | | | US | 2008194978 A1 | 14-08-2008 |
| | | | WO | 2005104937 A2 | 10-11-2005 |
| US 2002143265 | A1 | 03-10-2002 | NONE | | |
| US 2009088655 | A1 | 02-04-2009 | EP | 2186029 A2 | 19-05-2010 |
| | | | US | 2009088655 A1 | 02-04-2009 |
| | | | US | 2011118616 A1 | 19-05-2011 |
| | | | WO | 2009017820 A2 | 05-02-2009 |
| US 2007203418 | A1 | 30-08-2007 | US | 2007203418 A1 | 30-08-2007 |
| | | | WO | 2007098275 A2 | 30-08-2007 |

EPO FORM P0459

For more details about this annex : see Official Journal of the European Patent Office, No. 12/82

## REFERENCES CITED IN THE DESCRIPTION

**Non-patent literature cited in the description**

- **MACFARLANE PW et al.** The Early Repolarization Pattern: A Consensus Paper. *Journal of the American College of Cardiology*, 28 July 2015, vol. 66 (4), 470-77, https://doi.org/10.1016/j .j acc.2015.05.033
- **ERLING FALK** ; **PREDIMAN K. SHAH** ; **VALENTIN FUSTER**. Coronary Plaque Disruption. *Circulation*, 01 August 1995, vol. 92 (3), 657-71, https://doi.org/10.1161/01.CIR.92.3.657
- **EZRA A.** 2014 AHA/ACC Guideline for the Management of Patients With Non-ST-Elevation Acute Coronary Syndromes. *Journal of the American College of Cardiology*, 23 December 2014, vol. 64 (24), e139-228, https://doi.org/10.1016/j .j acc.2014.09.017
- **MILAD MATTA**. Stress Testing and Noninvasive Coronary Imaging: What's the Best Test for My Patient?. *Cleveland Clinic Journal of Medicine*, 01 September 2021, vol. 88 (9), 502-15, https://doi.org/10.3949/ccjm.88a.20068
- **TAYLOR DOWSLEY et al.** The Role of Noninvasive Imaging in Coronary Artery Disease Detection, Prognosis, and Clinical Decision Making. *Canadian Journal of Cardiology*, 01 March 2013, vol. 29 (3), 285-96, https://doi.org/10.1016/j.cjca.2012.10.022
- **ROBERT DETRANO** ; **RENATO GIANROSSI** ; **ICTOR FROELICHER**. The Diagnostic Accuracy of the Exercise Electrocardiogram: A Meta-Analysis of 22 Years of Research. *Progress in Cardiovascular Diseases*, 01 November 1989, vol. 32 (3), 173-206, https://doi.org/10.1016/0033-0620(89)90025-X
- **OBEYESEKERE MN** ; **KLEIN GJ** ; **NATTEL S** ; **LEONG-SIT P** ; **GULA LJ** ; **SKANES AC** ; **YEE R** ; **KRAHN AD**. A clinical approach to early repolarization. *Circulation.*, 16 April 2013, vol. 127 (15), 1620-9
- **HAYASHI, M.** ; **SHIMIZU, W.** ; **ALBERT, C.M.** The Spectrum of Epidemiology Underlying Sudden Cardiac Death. *Circ. Res.*, 2015, vol. 116, 1887-1906
- **GERBER, Y.** ; **JACOBSEN, S.J.** ; **FRYE, R.L.** ; **WESTON, S.A.** ; **KILLIAN, J.M.** ; **ROGER, V.L.** Secular trends in deaths from cardiovascular diseases: a 25-year community study. *Circulation*, 2006, vol. 113, 2285-2292
- **AZIZ, E.F.** ; **JAVED, F.** ; **PRATAP, B.** ; **HERZOG, E.** Strategies for the prevention and treatment of sudden cardiac death. *Open Access Emerg. Med. OAEM*, 2010, vol. 2010, 99-114
- **BURKE, A.P.** ; **FARB, A.** ; **MALCOM, G.T.** ; **LIANG, Y.H.** ; **SMIALEK, J.** ; **VIRMANI, R.** Coronary risk factors and plaque morphology in men with coronary disease who died suddenly. *N. Engl. J. Med.*, 1997, vol. 336, 1276-1282
- **FRIEDLANDER, Y.** ; **SISCOVICK, D.S.** ; **WEINMANN, S.** ; **AUSTIN, M.A.** ; **PSATY, B.M.** ; **LEMAITRE, R.N.** ; **ARBOGAST, P.** ; **RAGHUNATHAN, T.E.** ; **COBB, L.A.** Family history as a risk factor for primary cardiac arrest. *Circulation*, 1998, vol. 97, 155-160
- **RØE, A.T.** ; **FRISK, M.** ; **LOUCH, W.E.** Targeting cardiomyocyte Ca2+ homeostasis in heart failure. *Curr. Pharm. Des.*, 2015, vol. 21, 431-448
- **BERS, D.M.** Altered cardiac myocyte Ca regulation in heart failure. *Physiol. Bethesda Md*, 2006, vol. 21, 380-387
- **AMSTERDAM, E.A.** ; **WENGER, N.K.** ; **BRINDIS, R.G.** ; **CASEY, D.E.** ; **GANIATS, T.G.** ; **HOLMES, D.R.** ; **JAFFE, A.S.** ; **JNEID, H.** ; **KELLY, R.F.** ; **KONTOS, M.C.** 2014 AHA/ACC Guideline for the Management of Patients With Non-ST-Elevation Acute Coronary Syndromes. *Circulation*, 2014, vol. 130, e344-e426
- **KOLANSKY, DM**. Acute coronary syndromes: morbidity, mortality, and pharmacoeconomic burden. *Am J Manag Care*, March 2009, vol. 15 (2), 36-41
- **FISHER, R.A.** The Use of Multiple Measurements in Taxonomic Problems. *Ann. Eugen.*, 1936, vol. 7, 179-188
- **MOLINARO, A.M.** ; **SIMON, R.** ; **PFEIFFER, R.M.** Prediction error estimation: a comparison of resampling methods.. *Bioinformatics*, 2005, vol. 21, 3301-3307
- **FAY, M.P.** ; **PROSCHAN, M.A.** Wilcoxon-Mann-Whitney or t-test? On assumptions for hypothesis tests and multiple interpretations of decision rules. *Stat. Surv.*, 2010, vol. 4, 1-39
- **BEWICK, V.** ; **CHEEK, L.** ; **BALL, J.** Statistics review 12: Survival analysis. *Crit. Care*, 2004, vol. 8, 389-394

- **VINOGRADOVA, T.M.** ; **ZHOU, Y.Y.** ; **MALTSEV, V.** ; **LYASHKOV, A.** ; **STERN, M.** ; **LAKATTA, E.G.** Rhythmic ryanodine receptor Ca2+ releases during diastolic depolarization of sinoatrial pacemaker cells do not require membrane depolari-zation. *Circ. Res.*, 2004, vol. 94, 802-809